Europäisches Patentamt

(19) European Patent Office

Office européen des brevets

(11) Veröffentlichungsnummer: **0 020 962**
**B1**

(12) # EUROPÄISCHE PATENTSCHRIFT

(45) Veröffentlichungstag der Patentschrift:
22.12.82

(21) Anmeldenummer: 80102506.5

(22) Anmeldetag: 08.05.80

(51) Int. Cl.³: **B 01 J 23/66,** B 01 J 23/96,
C 07 D 301/10

(54) Verfahren zur Steigerung der Aktivität und zur Verlängerung der Standzeit bei hoher Selektivität von Silber-Träger-Katalysatoren für den Einsatz zur Ethylenoxid-Synthese.

(30) Priorität: 26.06.79 DE 2925625

(43) Veröffentlichungstag der Anmeldung:
07.01.81 Patentblatt 81/1

(45) Bekanntmachung des Hinweises auf die Patenterteilung:
22.12.82 Patentblatt 82/51

(84) Benannte Vertragsstaaten:
BE FR GB IT NL SE

(56) Entgegenhaltungen:
DE-A-2 753 359
US-A-4 012 425

Die Akte enthält technische Angaben, die nach dem Eingang der Anmeldung eingereicht wurden und die nicht in dieser Patentschrift enthalten sind.

(73) Patentinhaber: CHEMISCHE WERKE HÜLS AG,
Postfach 1320, D-4370 Marl 1 (DE)

(72) Erfinder: Vangermain, Erwin, Dr., Leverkusener Strasse 1, D-4370 Marl (DE)
Erfinder: Mengler, Claus-Dieter, Dr., Langehegge 147, D-4370 Marl (DE)
Erfinder: Elm, Rainer, Dr., Kampstrasse 102, D-4370 Marl (DE)
Erfinder: Ueberschaer, Horst, Bitterfelder Strasse 2, D-4370 Marl (DE)
Erfinder: Brauckmann, Wilhem, Sachsenstrasse 6, D-4370 Marl (DE)

## Verfahren zur Steigerung der Aktivität und zur Verlängerung der Standzeit bei hoher Selektivität von Silberträgerkatalysatoren für den Einsatz zur Ethylenoxidsynthese

Zur Herstellung von Ethylenoxid durch Oxidation von Ethylen mit Sauerstoff oder Sauerstoff enthaltenden Gasen werden Silberkatalysatoren eingesetzt. Es ist auch bekannt, derartige Katalysatoren mit sogenannten Promotoren zu versetzen, wobei insbesondere die Erdalkalimetallverbindungen, vorzugsweise Bariumverbindungen und/oder Alkalimetallverbindungen, insbesondere die der sogenannten schweren Alkalimetalle, Rubidium und/oder Cäsium, verwendet werden (deutsche Offenlegungsschriften Nrn. 2300512, 2733688, 2442568). Die Promotoren werden üblicherweise gleichzeitig mit dem Silber oder auch nach dem Silberaufzug auf den Träger bei der Herstellung des Katalysators aufgebracht.

Es ist auch bekannt, dass Silberkatalysatoren im Laufe der Zeit an Selektivität verlieren und nach einigen Jahren Gebrauch durch neuen Katalysator ersetzt werden müssen. Der Austausch eines in seiner Leistungsfähigkeit abgefallenen Katalysators durch einen neuen in grosstechnischen Anlagen, ist zeitraubend und arbeitsintensiv; er verursacht ausserdem Produktionsausfall und hohe Kosten.

Es ist auch bekannt, die Leistungsfähigkeit eines Silberkatalysators zu verbessern bzw. einen benutzten Katalysator zu reaktivieren (DE-A Nr. 2746976, DE-B Nr. 2519599).

Dies geschieht z.B. in Grossanlagen durch Fluten des mit dem Katalysator gefüllten Reaktors mit der Lösung der Promotormetallverbindung. Nach dem Abtrennen des überschüssigen Lösungsmittels wird durch Aufheizen und Durchblasen des Katalysatorbettes mit einem Inertgas das Restlösungsmittel entfernt. Diese Arbeitsweise erfordert das Anwenden von Lösungsmitteln und ebenfalls eine Produktionsunterbrechung.

Die gestellte Aufgabe besteht nun darin, durch eine einfache Methode Katalysatoren in ihrer Leistungsfähigkeit zu verbessern, ihre Leistungsfähigkeit möglichst lange zu erhalten, und den Austausch gegen einen neuen Katalysator möglichst weit hinzuschieben.

Die Lösung dieser Aufgabe gelingt, wenn man ein inniges Gemisch verwendet aus einem Silberträgerkatalysator und zu 0,5 bis 25 Volumenprozent, bezogen auf das Gesamtgemisch, einer Promotormetallverbindung oder einem mit Promotormetallverbindungen getränkten silberfreien Trägermaterial. Es ist demnach auch möglich, den Silberträgerkatalysator mit reinen Salzen der Promotormetalle zu mischen, wenn diese von der Form ihrer Kristalle her geeignet sind. Die verwendeten Träger für die Promotormetallverbindungen haben zweckmässigerweise die gleiche Form wie der Silberträgerkatalysator, weil damit eine gleichmässigere Mischung erreicht werden kann; dies ist jedoch keine Bedingung. Der Träger kann aus dem gleichen Grundmaterial bestehen wie der des Silberträgerkatalysators. Es können aber auch andere Trägermaterialien eingesetzt werden, soweit sie ausreichende mechanische Stabilität aufweisen und inert sind gegenüber den Einsatzstoffen sowie den Reaktionsprodukten des Oxidationsverfahrens. Geeignete Träger sind beispielsweise Bimsstein, Porzellan, Steinzeug, α-Aluminiumoxid, Stuttgarter Masse, ein Silikat, das geringe Mengen der Oxide des Natriums, Eisens, Calciums und Magnesiums enthält.

Die Menge der Promotormetalle, die in der Gesamtmischung enthalten ist, bewegt sich im üblichen Rahmen, nämlich 0,001 bis 0,05 Gew.% Rubidium, 0,001 bis 0,20 Gew.% Kalium, 0,001 bis 0,05 Gew.% Cäsium und 0,01 bis 0,25 Gew.% Barium, bezogen auf das Katalysatorgewicht.

Der Volumenanteil des die Promotormetalle enthaltenden Trägermaterials kann in weiten Grenzen variiert werden. Mit anderen Worten können auf den Träger auch relativ grosse Mengen der Promotormetalle aufgebracht werden, oder es können reine Promotormetallverbindungen in geeigneter äusserer Form eingesetzt werden. Der Volumenanteil des den Promotor enthaltenden Trägers an der Gesamtmischung soll den Wert von 50% nicht überschreiten, weil bei gegebenem Gesamtvolumen sonst der Silberanteil zu gering wird. Vorteilhaft liegt der Volumenanteil bei 0,5 bis 25%, insbesondere bei 5 bis 15%. Bei zu geringem Volumenanteil < 0,1% liegt keine ausreichende Verteilung in der Gesamtmenge vor.

Vorteilhaft verwendet man solche Verbindungen der Promotormetalle, die unter den Reaktionsbedingungen flüchtig sind.

Geeignete Promotormetallverbindungen sind insbesondere Bariumverbindungen, wie Bariumoxid, -peroxid, -hydroxid, -nitrit, -nitrat, -carbonat, -acetat, -oxalat, -tartrat, -naphthenat, -stearat, -dodecanat, -chlorid und/oder Alkalimetallverbindungen wie solche des Rubidiums und/oder Cäsiums, wie Oxide, Peroxide, Hydroxide, Nitrite, Nitrate, Carbonate, Acetate, Oxalate, Tartrate, Stearate, Dodecanate. Die Arbeitsweise ist sowohl anwendbar auf Silberkatalysatoren, die bereits die Promotoren enthalten, als auch auf solche Katalysatoren, die solche Promotoren nicht enthalten. In beiden Fällen wird die Leistungsfähigkeit der Katalysatoren gesteigert und über einen längeren Zeitraum erhalten.

Die folgenden Beispiele erläutern das Verfahren der Erfindung.

Der Versuchsreaktor besteht aus einem Reaktionsrohr von 6000 mm Länge und 26 mm Durchmesser aus rostfreiem Stahl. Es wurde jeweils ein Rohr mit einem in folgenden Beispielen näher beschriebenen Katalysator gefüllt. Ein zweites Rohr wurde mit jeweils dem gleichen Katalysator gefüllt, der vorher mit ca. 8% seines Volumens an Promotormetallverbindungen enthaltender Stuttgarter Masse vermischt worden war.

Die Rohre waren jeweils von einem Mantel umgeben, der Wasser bzw. Wasserdampf zum Abführen der Reaktionswärme enthielt. Die Katalysatoren enthielten jeweils 19,5 Gew.% Silber. Als Trägermaterial wurde ein Aluminiumoxidträger verwendet, der etwa 12 Gew.% Siliciumdioxid enthielt. Die Reaktionsrohre wurden gleichzeitig mit der gleichen Gasmischung betrieben. Diese Gasmischung hatte folgende Zusammensetzung:

25 Vol.% $C_2H_4$
49 Vol.% $CH_4$
6,8 Vol.% $O_2$
4,5 Vol.% $CO_2$
0,2 Vol.% $C_2H_6$
Rest $Ar + N_2$

*Beispiel 1:*

Es wurden 2,6 kg (2500 ml) des beschriebenen Silberkatalysators eingesetzt, der keine zusätzlichen Promotoren enthielt.

Das zweite Reaktionsrohr enthielt die gleiche Menge dieses Katalysators, der vor dem Einfüllen in das Rohr mit 200 ml Stuttgarter Masse vermischt worden war, die 1,1 g Kalium, 1,6 g Barium und 0,34 g Cäsium enthielt, die in Form einer wässrigen Lösung von Bariumperoxid, Kaliumcarbonat und Cäsiumnitrat aufgebracht worden waren.

Die Gasbelastung betrug in beiden Fällen nach Beendigung der Einlaufphase 9,26 Nm³/l Katalysator bei 19,8 bar Überdruck, entsprechend einer Gesamtgasbelastung von 25 Nm³/h. Der Chlorgehalt im Kreisgas wurde mit Hilfe von 1,2-Dichlorethan bei 7 bis 8 mg Cl/Nm³ gehalten. Die Dampfraumtemperatur wurde so reguliert, dass sich eine Ethylenoxidkonzentration von durchschnittlich 1,6 Vol.% im Reaktorausgang einstellte.

|  | Anfang | | Nach 6 Wochen Betriebszeit | |
|---|---|---|---|---|
|  | Selektiv. (Mol%) | Temp. (°C) | Selektiv. (Mol%) | Temp. (°C) |
| Vergleich | 74,0 | 254 | 71,6 | 271 |
| Erfindung | 74,2 | 252 | 77,1 | 245 |

*Beispiel 2:*

Es wurde als Vergleichskatalysator ein solcher verwendet, der bereits 472 ppm Barium enthielt. Unter sonst gleichen Bedingungen wurden folgende Ergebnisse erhalten:

|  | Anfang | | Nach 6 Wochen Betriebszeit | |
|---|---|---|---|---|
|  | Selektiv. (Mol%) | Temp. (°C) | Selektiv. (Mol%) | Temp. (°C) |
| Vergleich | 75,7 | 249 | 73,5 | 267 |
| Erfindung | 75,8 | 249 | 77,9 | 245 |

*Beispiel 3:*

Es wurde als Vergleichskatalysator ein solcher verwendet, der bereits 481 ppm Barium und 91 ppm Cäsium enthielt. Unter sonst gleichen Bedingungen wurden folgende Ergebnisse erzielt:

|  | Anfang | | Nach 6 Wochen Betriebszeit | |
|---|---|---|---|---|
|  | Selektiv. (Mol%) | Temp. (°C) | Selektiv. (Mol%) | Temp. (°C) |
| Vergleich | 80,4 | 245 | 77,5 | 265 |
| Erfindung | 80,3 | 245 | 81,5 | 242 |

*Beispiel 4:*

Als Vergleichskatalysator wurde wie in Beispiel 1 ein reiner Silberkatalysator eingesetzt. Erfindungsgemäss enthielt die Trägerschicht anstelle von Cäsium 0,29 g Rubidium. Folgende Ergebnisse wurden erhalten:

|  | Anfang | | Nach 6 Wochen Betriebszeit | |
|---|---|---|---|---|
|  | Selektiv. (Mol%) | Temp. (°C) | Selektiv. (Mol%) | Temp. (°C) |
| Vergleich | 74,0 | 254 | 71,6 | 271 |
| Erfindung | 74,2 | 252 | 74,9 | 251 |

## Patentansprüche

1. Verfahren zur Steigerung der Aktivität und zur Verlängerung der Standzeit bei hoher Selektivität von Silberträgerkatalysatoren für die Herstellung von Ethylenoxid durch Oxidation von Ethylen mit Sauerstoff oder Sauerstoff enthaltenden Gasen mit Hilfe von Promotormetallen, dadurch gekennzeichnet, dass ein Gemisch verwendet wird aus einem bekannten Silberträgerkatalysator und zu 0,5 bis 25 Vol.%, bezogen auf das Gesamtgemisch, einer Promotormetallverbindung oder einem mit Promotormetallverbindungen getränkten silberfreien Trägermaterial.

2. Verfahren nach Anspruch 1, dadurch gekennzeichnet, dass als Verbindungen der Promotormetalle Verbindungen des Kaliums und/oder Rubidiums und/oder Cäsiums und/oder Bariums verwendet werden.

## Revendications

1. Procédé pour augmenter l'activité et pour prolonger le temps de maintien, à haute sélectivité, de catalyseurs à l'argent sur support pour la préparation d'oxyde d'éthylène avec de l'oxygène ou des gaz renfermant de l'oxygène, à l'aide de métaux promoteurs, caractérisé par le fait qu'on utilise un mélange constitué par un catalyseur connu à l'argent sur support et pour 0,5 à 25% en volume, relativement au mélange total, par un composé d'un métal promoteur ou par une matière de support exempte d'argent qui est imbibée de composés de métaux promoteurs.

2. Procédé selon la revendication 1, caractérisé par le fait que, comme composés des métaux promoteurs, on utilise des composés du potassium et/ou du rubidium et/ou du césium et/ou du baryum.

## Claims

1. A process for increasing the activity, and prolonging the lifetime at high selectivity, of a supported silver catalyst for the manufacture of ethylene oxide by oxidation of ethylene with oxygen or an oxygen-containing gas with the aid of a promoter metal, characterized in that a mixture is used of a known supported silver catalyst and 0.5 to 25% by volume, based on the total mixture, of a promoter metal compound or of a silver-free support impregnated with a promoter metal compound.

2. A process according to claim 1, characterized in that a compound of potassium and/or a compound of rubidium and/or a compound of caesium and/or a compound of barium is used as the promoter metal compound.